# EUROPEAN PATENT SPECIFICATION

(11) **EP 2 285 841 B1**
(45) Date of publication and mention of the grant of the patent: **14.11.2012**
(21) Application number: 09743720.6
(22) Date of filing: 08.05.2009
(51) Int. Cl.: C08F 14/18, C08K 5/06, C08K 5/095, C08F 6/16, C08F 2/16, B01F 17/42, C08F 214/18

(54) **ABATEMENT OF FLUOROETHER CARBOXYLIC ACIDS OR SALTS EMPLOYED IN FLUOROPOLYMER RESIN MANUFACTURE**
VERRINGERUNG DES GEHALTS AN FLUORETHERCARBONSÄUREN ODER DEREN SALZEN, DIE BEI DER HERSTELLUNG VON FLUORPOLYMERHARZEN EINGESETZT WERDEN
RÉDUCTION DE LA TENEUR EN ACIDES FLUOROÉTHERS CARBOXYLIQUES OU DE LEURS SELS UTILISÉS LORS DE LA FABRICATION DE RÉSINES DE FLUOROPOLYMÈRES

(30) Priority: 09.05.2008 US 51898 P
(43) Date of publication of application: 23.02.2011
(73) Proprietor: E. I. Du Pont de Nemours and Company, Wilmington, DE 19898 (US)
(72) Inventor: BROTHERS, Paul, Douglas, Chadds Ford, PA 19317 (US); LEUNG, Lam, H., Bear, DE 19701 (US); GANGAL, Subhash, Vishnu, Hockessin, DE 19707 (US)
(74) Representative: Matthews, Derek Peter
(86) International application number: PCT/US2009/043222
(87) International publication number: WO 2009/137734

(56) References cited:
- EP-A- 0 816 397
- EP-A- 0 822 175
- EP-A- 1 845 116
- WO-A-2005/121290
- US-A- 5 969 192
- US-A1- 2007 015 864

## Description

### FIELD OF THE INVENTION

This invention relates to a process for making fluoropolymer resin by dispersion polymerization of fluorinated monomer in an aqueous medium in the presence of fluoroether carboxylic acid or salt which includes abatement of the fluoroether carboxylic acid or salt.

### BACKGROUND OF THE INVENTION

A typical process for the aqueous dispersion polymerization of fluorinated monomer includes feeding fluorinated monomer to a heated reactor containing a fluorosurfactant and deionized water. Paraffin wax is employed in the reactor as a stabilizer for some polymerizations, e.g., polytetrafluoroethylene (PTFE) homopolymers. A free-radical initiator solution is employed and, as the polymerization proceeds, additional fluorinated monomer is added to maintain the pressure. A chain transfer agent is employed in the polymerization of some polymers, e.g., melt-processible TFE copolymers to control melt viscosity. After several hours, the feeds are stopped, the reactor is vented and purged with nitrogen, and the raw dispersion in the vessel is transferred to a cooling vessel.

For use in fluoropolymer coatings for metals, glass and fabric, polymer dispersion is typically transferred to a dispersion concentration operation which produces stabilized dispersions used as coatings. Certain grades of PTFE dispersion are made for the production of fine powder. For this use, the polymer dispersion is coagulated, the aqueous medium is removed and the PTFE is dried to produce fine powder. Dispersions of melt-processible fluoropolymers for molding resin use are also coagulated and the coagulated polymer dried and then processed into a convenient form such as flake, chip or pellet for use in subsequent melt-processing operations.

Because of recent environmental concerns with regard to perfluorooctanoic acid and salts, there is interest in reducing or eliminating perfluorooctanoic acid and salts in fluoropolymer polymerization processes. Recently, there is an interest in using fluoroether carboxylic acids or salts in place of perfluoroalkane carboxylic acids or salts in the polymerization of fluoropolymers. In addition, fluoropolymer manufacturers desire to recycle or otherwise contain fluoroether carboxylic acids or salts used in fluoropolymer manufacturing. For example, US 2007/0015937 A1 discloses the use of fluoroether carboxylic acid surfactant in fluoropolymerization and its recycle from certain waste streams. The fluoroether carboxylic acid surfactants of US 2007/0015937 A1 have the formula:

[R_{f}-O-L-COO⁻]ᵢ Xⁱ⁺

wherein L represents a linear partially or fully fluorinated alkylene group or an aliphatic hydrocarbon group, R_{f} represents a linear partially or fully fluorinated aliphatic group or a linear partially or fully fluorinated aliphatic group interrupted with one or more oxygen atoms, and Xⁱ⁺ represents a cation having a valence i and i is 1, 2, or 3. Surfactants of this formula are referred to hereinafter as "linear fluoroether carboxylic acids or salts".

For the manufacture of fluoropolymer resins in which the fluoropolymer resin is isolated and dried, a substantial portion of the fluorinated surfactant remains with the wet fluoropolymer isolated from the aqueous medium by coagulation. Consequently, when the fluoropolymer is heated for the purposes of drying, the fluorosurfactant is present in the drier exhaust stream and will escape into the environment unless captured. US 2007/0015937 A1 proposes capturing the linear fluoroether surfactants used in that process from the exhaust gas by use of an aqueous scrubber to produce an aqueous solution containing the linear fluoroether surfactant. However, as disclosed in US 2007/0015937 A1, recycling of linear fluoroether surfactant from this solution is complicated and expensive. In one embodiment, the linear fluoroether surfactant is first removed from the aqueous solution by treatment with absorbent particles such as anion exchange resins. Then, the linear fluoroether surfactant is eluted from the anion exchange resin which requires any of a variety of specially formulated eluting compositions which may contain salts, organic solvents, bases, etc. Alternatively, absorbent particles containing the surfactant are contacted with an alcohol to convert the linear fluoroether surfactant to an ester derivative which is then distilled. This alternative also requires a subsequent conversion back to salt by saponification, typically with ammonia. US 2007/0015937 A1 also discloses that when linear carboxylic acid fluorosurfactants are recovered, the desired high purity to enable reuse in aqueous fluoropolymerization requires purification treatment such as treatment at elevated temperatures with oxidants such as dichromates, peroxodisulfates or permanganates followed by pure product isolation by crystallization or reduced pressure distillation.

A process for the manufacture of fluoropolymer resin by dispersion polymerization of fluorinated monomer in an aqueous medium in the presence of fluoroether carboxylic acid or salt is desired which simplifies abatement of fluoroether carboxylic acids or salts.

### SUMMARY OF THE INVENTION

The invention provides a process for making fluoropolymer resin comprising polymerizing at least one fluorinated monomer in an aqueous medium containing initiator and polymerization agent comprising fluoroether carboxylic acid or salt thereof to form an aqueous dispersion of particles of fluoropolymer. The fluoroether carboxylic acid or salt thereof employed in the process has the structure indicated by Formula I below:

[R¹-O-L-COO⁻] Y⁺ (I)

wherein R¹ is a linear, branched or cyclic partially or fully fluorinated aliphatic group which may contain ether linkages; L is a branched partially or fully fluorinated alkylene group which may contain ether linkages; and Y⁺ is hydrogen, ammonium or alkali metal cation. Fluoroethers of this formula are referred to hereinafter as "branched fluoroether carboxylic acid or salt". Wet fluoropolymer resin is isolated from the aqueous medium with the wet fluoropolymer resin containing residual fluoroether carboxylic acid or salt. The wet fluoropolymer resin is heated to remove water to produce a dry fluoropolymer resin and to decarboxylate the residual fluoroether carboxylic acid or salt to produce a vapor of fluoroether byproduct. The vapor of fluoroether byproduct is captured.

The invention is based on the recognition that branched fluoroether carboxylic acid or salt can be decarboxylated to produce fluoroether byproducts at lower temperatures than linear fluoroether carboxylic acid or salt and that decarboxylation and capture of the fluoroether byproducts can be employed for abatement purposes. Accordingly, high amounts of branched fluoroether carboxylic acid or salt are decarboxylated and converted to fluoroether byproduct during heating to dry the wet polymer and make capture of fluoroether byproducts an effective method for abatement of the fluoroether carboxylic acid or salt. In an embodiment of the invention, the fluoroether byproduct is captured in a bed of adsorbent particles, preferably activated carbon. In accordance with another embodiment of the invention, fluoroether byproduct is recovered, preferably by thermally desorbing the fluoroether byproduct from the adsorbent particles.

### DETAILED DESCRIPTION OF THE INVENTION

### Polymerization Agent

Any of a wide variety of branched fluoroether carboxylic acids or salts according to Formula I above can be employed as polymerization agent in a process in accordance with the invention. Preferably, L in Formula I is a branched fully fluorinated alkylene group which may contain ether linkages and more preferably is -CF(CF₃)-. A single compound can be employed as polymerization agent or mixture of two or more compounds can be employed. If a single branched fluoroether carboxylic acid or salt is used, it typically will have properties such as molecular weight, surface tension and solubility similar to that of perfluorooctanoic acid or salt. Such branched fluoroether carboxylic acids or salts can be fluoromonoether carboxylic acids or salts or low molecular weight fluoropolyether carboxylic acids or salts. Mixtures of two or more of such surfactants can be used if desired. As will be described in more detail hereinafter, a preferred embodiment employs a mixture of a low molecular weight branched fluoroether carboxylic acid or salt, which has properties similar to a conventional fluorosurfactant, and a high molecular weight branched fluoroether carboxylic acid or salt, e.g., a branched fluoropolyether carboxylic acid or salt which has a molecular weight higher than the typical range and therefore different properties compared to a conventional fluorosurfactant. As will become apparent in the following, the higher molecular weight branched fluoropolyether carboxylic acid or salt is advantageously provided as a mixture of branched fluoropolyether carboxylic acids or salts which has particular molecular weight fractions which provide for efficient use in polymerization.

In accordance with a preferred form of the invention, the fluoroether carboxylic acid or salt is highly fluorinated. "Highly fluorinated" means that at least about 50% of the total number of fluorine and hydrogen atoms in the fluorosurfactant are fluorine atoms. More preferably, at least about 75% of the total number of fluorine and hydrogen atoms in the fluoroether carboxylic acid or salt are fluorine atoms, most preferably at least about 90%. Perfluoroether carboxylic acid or salt is also preferred for use in accordance with the invention.

In a preferred embodiment of the invention, the branched fluoroether carboxylic acid or salt is a compound or mixtures of compounds having a structure represented by formula (I) wherein R¹ is CF₃-CF₂-CF₂-O-(-CFCF₃-CF₂-O-)ₙ, n is 0-35 and L is -CF(CF₃)-. For convenience, these compounds may be represented by Formula II:

[CF₃-CF₂-CF₂-O-(-CFCF₃-CF₂-O-)ₙ-CFCF₃-COO⁻] Y⁺ (II)

wherein n is 0-35 and Y⁺ is hydrogen, ammonium or alkali metal cation. Preferably, Y⁺ is hydrogen or ammonium.

In one preferred embodiment of the invention, a mixture of branched fluoropolyether carboxylic acids or salts having a number average molecular weight of at least about 800 g/mol is employed in combination with a branched fluoroether carboxylic acid or salt surfactant. Preferably, the branched fluoroether carboxylic acid or salt surfactant has a chain length of no greater than 6. "Chain length" as used in this application refers to the number of atoms in the longest linear chain in the hydrophobic tail of the fluoroether employed in the process of this invention, i.e., the portion of the structure in Formula I represented by R¹-O-L-. Chain length includes atoms such as oxygen atoms in addition to carbon in the chain of hydrophobic tail of the surfactant but does not include branches of the longest linear chain or include the carbon atom of the carboxylate group. Preferably, the chain length is 4 to 6 atoms. In accordance with another preferred form of the invention the chain length is 3 to 5 atoms. Most preferably, the chain length of is 4 to 5 atoms.

For example, within the scope of the preferred branched fluoroether carboxylic acids or salts of Formula II above, a suitable compound to be used in this form of the invention in combination with fluoropolyether carboxylic acid or salt having a number average molecular weight of at least about 800 g/mol is provided by n being equal to 0, and is represented by Formula III below:

[CF₃CF₂CF₂OCF(CF₃)COO⁻] Y⁺ (III)

wherein Y⁺ is hydrogen, ammonium, or alkali metal cation. This compound is referred to hereinafter as dimer acid (DA) in acid form and dimer acid salt (DAS) in salt form. The chain length of this compound is 5. A compound of this formula can be obtained from the perfluoro-2-propoxypropionyl fluoride intermediate prepared according U.S. Patent 3,291,843 or dimerization of hexafluoropropylene oxide and subsequent hydrolysis of the resulting acyl fluoride to carboxylic acid in the case of the acid and, in the case of the salt, by simultaneous or subsequent reaction with the appropriate base to produce the desired salt. A procedure for dimerization of hexafluoropropylene oxide is disclosed in G.B. Patent 1 292 268.

In the preferred form of the invention employing a branched fluoropolyether carboxylic acid or salt having a number average molecular weight of at least about 800 g/mol in combination with a branched fluoroether carboxylic acid or salt surfactant, a mixture of branched fluoropolyether carboxylic acids or salts preferably is employed which has a composition represented by Formula II above wherein Y⁺ is hydrogen, ammonium or alkali metal cation, wherein n is at least 1 and has an average value of about 3 to about 13 (number average molecular weight of about 800 to about 2500 g/mol). In this preferred mixture, the amount of perfluoropolyether acid or salt wherein n is 1 is not more than 50 ppm by weight of the total amount of perfluoropolyethers in the mixture. The amount of perfluoropolyethers in the mixture wherein n is 13 or greater is not more than 40% by weight of the total amount of perfluoropolyethers in said mixture. In preferred embodiments of the invention, the amount of perfluoropolyethers in the mixture where n is greater than 13 is not more than 35% by weight, not more than 30% by weight, not more than 20% by weight, not more than 10% by weight and not more than 7.5%. In preferred embodiments, Y⁺ is hydrogen or ammonium.

In another embodiment of the invention the amount of perfluoropolyethers in the mixture wherein n is 16 or greater is not more than 10% by weight of the total amount of perfluoropolyethers in the mixture. In preferred embodiments of the invention, the amount of perfluoropolyethers in the mixture where n is 16 or greater is not more than 7 % by weight, not more than 5% by weight, and not more than 3% by weight.

In a further embodiment of the invention the amount of perfluoropolyethers in the mixture wherein n is 4 or less is not more than 10% by weight of the total amount of perfluoropolyethers in the mixture, more preferably not more than 1% by weight of the total amount of perfluoropolyethers in the mixture.

In yet another embodiment of the invention, at least about 50% by weight of the perfluoropolyethers in the mixture fall within the range of n=3 to n=13. In other embodiments of the invention, at least about 60% by weight of the perfluoropolyethers in the mixture fall within the range of n=3 to n=13, preferably 75%, and more preferably 90% by weight of the perfluoropolyethers.

In further embodiments of the invention, the composition comprises a mixture of perfluoropolyether acids or salts of formula I wherein n has an average value of about 4 to about 13 (number average molecular weight of about 1000 to about 2500 g/mol), preferably an average value of about 5 to about 13 (number average molecular weight of about 1150 to about 2500 g/mol), more preferably an average value of about 5 to about 10 (number average molecular weight of about 1150 to about 1700 g/mol).

A mixture of perfluoropolyether acids or salt can be prepared by the polymerization of hexafluoropropylene oxide (HFPO) forming the perfluoropolyether acyl fluoride. The reaction product of the polymerization of hexafluoropropylene oxide is a mixture of perfluoropolyethers of varying degree of polymerization resulting in a distribution of various molecular weight oligomers. Low molecular weight oligomers are separated by distillation and recycled. In a preferred embodiment, the acyl fluoride can be hydrolyzed to carboxylic acid and converted to the salt if desired by use of the appropriate base such as ammonium hydroxide to form the ammonium salt.

The mixture of perfluoropolyether carboxylic acids or salts according to the present invention with the limits stated above on the amount of both low molecular weight and high molecular weight fractions can be obtained via fractional distillation of the acyl fluoride.

In the preferred form of the invention employing a polymerizing agent comprising a branched fluoropolyether carboxylic acid or salt having a number average molecular weight of at least about 800 g/mol in combination with a branched fluoroether carboxylic acid or salt surfactant, the ratio in the polymerization agent of the weight of the fluorosurfactant to the weight of the mixture of perfluoropolyether carboxylic acids or salts is about 2:1 to about 200:1. In other embodiments of the invention, the ratio of the weight of said fluorosurfactant to the weight of said mixture of perfluoropolyether acids or salts is about 3:1 to about 150:1, preferably about 5:1 to about 100:1, more preferably 10:1 to about 80:1.

In a preferred polymerization process in accordance with the invention, the amount of the mixture of fluoropolyether acid or salt having a number average molecular weight of at least about 800 g/mol employed as polymerization agent in the aqueous polymerization medium preferably is present in the range of about 5 to about 3,000 ppm based on the weight of water in the aqueous polymerization medium.

To form the preferred polymerization agent comprising a branched fluoropolyether carboxylic acid or salt having a number average molecular weight of at least about 800 g/mol in combination with a branched fluoroether carboxylic acid or salt surfactant, the mixture of branch perfluoroether carboxylic acid or salt and branched fluoroether carboxylic acid or salt surfactant are preferably dispersed adequately in aqueous medium to function effectively as a polymerization agent. "Dispersed" as used in this application refers to either dissolved in cases in which the mixture of branched fluoropolyether carboxylic acid or salt and branched fluoroether carboxylic acid or salt surfactant are soluble in the aqueous medium or dispersed in cases in which the mixture of fluoropolyether carboxylic acid or salt and/or the fluoroether carboxylic acid or salt surfactant are not fully soluble and are present in very small particles, for example about 1 nm to about 1 µm, in the aqueous medium. Similarly, "dispersing" as used in this application refers to either dissolving or dispersing the mixture of branched fluoropolyether acid or salt and/or the branched fluoroether carboxylic acid or salt surfactant so that they are dispersed as defined above. Preferably, the mixture of branched fluoropolyether carboxylic acids or salts and branched fluoroether carboxylic acid or salt surfactant are dispersed sufficiently so that the polymerization medium containing the mixture of branched fluoropolyether carboxylic acids or salts and branched fluoroether carboxylic acid or salt surfactant appears water clear or nearly water clear. Clarity of the mixture is typically an indicator of improved polymerization performance, e.g., polymerizations employing mixtures of lower haze typically produce less undispersed polymer (coagulum) than mixtures with higher haze values.

Dispersing of the mixture of branched fluoropolyether carboxylic acids or salts and the branched fluoroether carboxylic acid or salt surfactant can be carried out by a variety of methods. In one suitable procedure, the polymerization agent can be made directly in the aqueous polymerization medium. In this procedure, the mixture of branched fluoropolyether carboxylic acids or salts is supplied in acid form and subsequently converted to salt form. This is accomplished by first adding ammonium hydroxide or alkali metal hydroxide, preferably ammonium hydroxide, to the aqueous polymerization medium in a quantity sufficient to substantially completely convert to salt form the subsequently added fluoropolyether carboxylic acid mixture. The branched fluoropolyether carboxylic acid can then be added to the ammonium hydroxide or alkali metal hydroxide solution and, if desired, pH measurements can be made to determine if insufficient or excess base has been used. In addition, as known in the art, the amount of ammonium hydroxide or alkali metal hydroxide used, together with other materials added to the polymerization medium, should provide a pH in the aqueous polymerization medium which promotes the desired level of activity for the particular initiator system used and provides an operable pH range for the polymerization agent. The branched fluoroether carboxylic acid or salt surfactant can be added to the aqueous polymerization medium prior to, simultaneously with or subsequently to the addition of the mixture of branched fluoropolyether carboxylic acid.

In a preferred embodiment, the branched fluoroether carboxylic acid or salt and the mixture of branched fluoropolyether carboxylic acids or salts are both supplied in acid form. It has been discovered that the mixture of branched fluoropolyether carboxylic acids and branched fluoropolyether carboxylic acid fluorosurfactant will form a mixture which can be converted to salt form to make a concentrate in which the branched fluoropolyether carboxylate salt is dispersed. The concentrate is advantageously used to provide the branched fluoroether carboxylic acid or salt surfactant and the mixture of branched fluoropolyether carboxylic acids or salts to the aqueous medium in dispersed form.

### Initiators

Polymerization in accordance with the invention employs free radical initiators capable of generating radicals under the conditions of polymerization. As is well known in the art, initiators for use in accordance with the invention are selected based on the type of fluoropolymer and the desired properties to be obtained, e.g., end group type, molecular weight, etc. For some fluoropolymers such as melt-processible TFE copolymers, water-soluble salts of inorganic peracids are employed which produce anionic end groups in the polymer. Preferred initiators of this type have a relatively long half-life, preferably persulfate salts, e.g., ammonium persulfate or potassium persulfate. To shorten the half-life of persulfate initiators, reducing agents such as ammonium bisulfite or sodium metabisulfite, with or without metal catalyst salts such as iron, can be used. Preferred persulfate initiators are substantially free of metal ions and most preferably are ammonium salts.

For the production of PTFE or modified PTFE dispersions for dispersion end uses, small amounts of short chain dicarboxylic acids such as succinic acid or initiators that produce succinic acid such as disuccinic acid peroxide (DSP) are preferably also added in addition to the relatively long half-life initiators such as persulfate salts. Such short chain dicarboxylic acids are typically beneficial in reducing undispersed polymer (coagulum). For the production of PTFE dispersion for the manufacture of fine powder, a redox initiator system such as potassium permanganate/oxalic acid is often used.

The initiator is added to the aqueous polymerization medium in an amount sufficient to initiate and maintain the polymerization reaction at a desired reaction rate. At least a portion of the initiator is preferably added at the beginning of the polymerization. A variety of modes of addition may be used including continuously throughout the polymerization, or in doses or intervals at predetermined times during the polymerization. A particularly preferred mode of operation is for initiator to be precharged to the reactor and additional initiator to be continuously fed into the reactor as the polymerization proceeds. Preferably, total amounts of ammonium persulfate and/or potassium persulfate employed during the course of polymerization are about 25 ppm to about 250 ppm based on the weight of the aqueous medium. Other types of initiators, for example, potassium permanganate/oxalic acid initiators, can be employed in amounts and in accordance with procedures as known in the art.

### Chain Transfer Agents

Chain-transfer agents may be used in a process in accordance with the invention for the polymerization of some types of polymers, e.g., for melt-processible TFE copolymers, to decrease molecular weight for the purposes of controlling melt viscosity. Chain transfer agents useful for this purpose are well-known for use in the polymerization of fluorinated monomers. Preferred chain transfer agents include hydrogen, aliphatic hydrocarbons, halocarbons, hydrohalocarbons or alcohol having 1 to 20 carbon atoms, more preferably 1 to 8 carbon atoms. Representative preferable examples of such chain transfer agents are alkanes such as ethane, chloroform, 1,4-diiodoperfluorobutane and methanol.

The amount of a chain transfer agent and the mode of addition depend on the activity of the particular chain transfer agent and on the desired molecular weight of the polymer product. A variety of modes of addition may be used including a single addition before the start of polymerization, continuously throughout the polymerization, or in doses or intervals at predetermined times during the polymerization. The amount of chain train transfer agent supplied to the polymerization reactor is preferably about 0.005 to about 5 wt%, more preferably from about 0.01 to about 2 wt% based upon the weight of the resulting fluoropolymer.

### Fluoropolymer

Fluoropolymer dispersions formed by this invention are comprised of particles of fluoropolymer made from at least one fluorinated monomer, i.e., wherein at least one of the monomers contains fluorine, preferably an olefinic monomer with at least one fluorine or a perfluoroalkyl group attached to a doubly-bonded carbon. The fluorinated monomer used in the process of this invention is preferably independently selected from the group consisting of tetrafluoroethylene (TFE), hexafluoropropylene (HFP), chlorotrifluoroethylene (CTFE), trifluoroethylene, hexafluoroisobutylene, perfluoroalkyl ethylene, fluorovinyl ethers, vinyl fluoride (VF), vinylidene fluoride (VF2), perfluoro-2,2-dimethyl-1,3-dioxole (PDD), perfluoro-2-methylene-4-methyl-1,3-dioxolane (PMD), perfluoro(allyl vinyl ether) and perfluoro(butenyl vinyl ether). A preferred perfluoroalkyl ethylene monomer is perfluorobutyl ethylene (PFBE). Preferred fluorovinyl ethers include perfluoro(alkyl vinyl ether) monomers (PAVE) such as perfluoro(propyl vinyl ether) (PPVE), perfluoro(ethyl vinyl ether) (PEVE), and perfluoro(methyl vinyl ether) (PMVE). Non-fluorinated olefinic comonomers such as ethylene and propylene can be copolymerized with fluorinated monomers.

Fluorovinyl ethers also include those useful for introducing functionality into fluoropolymers. These include CF₂=CF-(O-CF₂CFR_{f})ₐ-O-CF₂CFR'_{f}SO₂F, wherein R_{f} and R'_{f} are independently selected from F, Cl or a perfluorinated alkyl group having 1 to 10 carbon atoms, a = 0, 1 or 2. Fluorovinyl ethers of this type are disclosed in U.S. Patent No. 3,282,875 (CF₂=CF-O-CF₂CF(CF₃)-O-CF₂CF₂SO₂F, perfluoro(3,6-dioxa-4-methyl-7-octenesulfonyl fluoride)), and in U.S. Patent Nos. 4,358,545 and 4,940,525 (CF₂=CF-O-CF₂CF₂SO₂F). Another example is CF₂=CF-O-CF₂-CF(CF₃)-O-CF₂CF₂CO₂CH₃, methyl ester of perfluoro(4,7-dioxa-5-methyl-8-nonenecarboxylic acid), disclosed in U.S. Patent No. 4,552,631. Similar fluorovinyl ethers with functionality of nitrile, cyanate, carbamate, and phosphonic acid are disclosed in U.S. Patent Nos. 5,637,748; 6,300,445; and 6,177,196.

The invention is especially useful when producing dispersions of polytetrafluoroethylene (PTFE) including modified PTFE. PTFE and modified PTFE typically have a melt creep viscosity of at least about 1 x 10⁸ Pa·s and, with such high melt viscosity, the polymer does not flow significantly in the molten state and therefore is not a melt-processible polymer. Polytetrafluoroethylene (PTFE) refers to the polymerized tetrafluoroethylene by itself without any significant comonomer present. Modified PTFE refers to copolymers of TFE with such small concentrations of comonomer that the melting point of the resultant polymer is not substantially reduced below that of PTFE. The concentration of such comonomer is preferably less than 1 wt%, more preferably less than 0.5 wt%. A minimum amount of at least about 0.05 wt% is preferably used to have significant effect. The small amount of comonomer modifier improves film forming capability during baking (fusing). Comonomer modifiers include perfluoroolefin, notably hexafluoropropylene (HFP) or perfluoro(alkyl vinyl ether) (PAVE), where the alkyl group contains 1 to 5 carbon atoms, with perfluoro(ethyl vinyl ether) (PEVE) and perfluoro(propyl vinyl ether) (PPVE) being preferred. Chlorotrifluoroethylene (CTFE), perfluorobutyl ethylene (PFBE), or other monomer that introduces bulky side groups into the molecule are also possible comonomer modifiers.

The invention is especially useful when producing dispersions of melt-processible fluoropolymers. By melt-processible, it is meant that the polymer can be processed in the molten state (i.e., fabricated from the melt into shaped articles such as films, fibers, and tubes etc. that exhibit sufficient strength and toughness to be useful for their intended purpose) using conventional processing equipment such as extruders and injection molding machines. Examples of such melt-processible fluoropolymers include homopolymers such as polychlorotrifluoroethylene or copolymers of tetrafluoroethylene (TFE) and at least one fluorinated copolymerizable monomer (comonomer) present in the polymer usually in sufficient amount to reduce the melting point of the copolymer substantially below that of TFE homopolymer, polytetrafluoroethylene (PTFE), e.g., to a melting temperature no greater than 315°C.

A melt-processible TFE copolymer typically incorporates an amount of comonomer into the copolymer in order to provide a copolymer which has a melt flow rate (MFR) of about 1-100 g/10 min as measured according to ASTM D-1238 at the temperature which is standard for the specific copolymer. Preferably, the melt viscosity is at least about 10² Pa·s, more preferably, will range from about 10² Pa·s to about 10⁶ Pa·s, most preferably about 10³ to about 10⁵ Pa·s measured at 372°C by the method of ASTM D-1238 modified as described in U.S. Patent 4,380,618. Additional melt-processible fluoropolymers are the copolymers of ethylene (E) or propylene (P) with TFE or CTFE, notably ETFE, ECTFE and PCTFE.

A preferred melt-processible copolymer for use in the practice of the present invention comprises at least about 40-98 mol% tetrafluoroethylene units and about 2-60 mol% of at least one other monomer. Preferred comonomers with TFE are perfluoroolefin having 3 to 8 carbon atoms, such as hexafluoropropylene (HFP), and/or perfluoro(alkyl vinyl ether) (PAVE) in which the linear or branched alkyl group contains 1 to 5 carbon atoms. Preferred PAVE monomers are those in which the alkyl group contains 1, 2, 3 or 4 carbon atoms, and the copolymer can be made using several PAVE monomers. Preferred TFE copolymers include FEP (TFE/HFP copolymer), PFA (TFE/PAVE copolymer), TFE/HFP/PAVE wherein PAVE is PEVE and/or PPVE, MFA (TFE/PMVE/PAVE wherein the alkyl group of PAVE has at least two carbon atoms) and THV (TFE/HFP/VF2).

Further useful polymers are film forming polymers of polyvinylidene fluoride (PVDF) and copolymers of vinylidene fluoride as well as polyvinyl fluoride (PVF) and copolymers of vinyl fluoride.

The invention is also useful when producing dispersions of fluorocarbon elastomers. These elastomers typically have a glass transition temperature below 25°C and exhibit little or no crystallinity, i.e. are amorphous at room temperature. Fluorocarbon elastomer copolymers made by the process of this invention typically contain 25 to 70 wt%, based on total weight of the fluorocarbon elastomer, of copolymerized units of a first fluorinated monomer which may be vinylidene fluoride (VF2) or tetrafluoroethylene (TFE). The remaining units in the fluorocarbon elastomers are comprised of one or more additional copolymerized monomers, different from the first monomer, selected from the group consisting of fluorinated monomers, hydrocarbon olefins and mixtures thereof. Fluorocarbon elastomers prepared by the process of the present invention may also, optionally, comprise units of one or more cure site monomers. When present, copolymerized cure site monomers are typically at a level of 0.05 to 7 wt%, based on total weight of fluorocarbon elastomer. Examples of suitable cure site monomers include: i) bromine -, iodine -, or chlorine - containing fluorinated olefins or fluorinated vinyl ethers; ii) nitrile group-containing fluorinated olefins or fluorinated vinyl ethers; iii) perfluoro(2-phenoxypropyl vinyl ether); and iv) non-conjugated dienes.

Preferred TFE based fluorocarbon elastomer copolymers include TFE/PMVE, TFE/PMVE/E, TFE/P and TFE/P/VF2. Preferred VF2 based fluorocarbon elastomer copolymers include VF2/HFP, VF2/HFP/TFE, and VF2/PMVE/TFE. Any of these elastomer copolymers may further comprise units of cure site monomer.

### Process

In the practice of a preferred embodiment of the invention, the process is carried out as a batch process in a pressured reactor. Suitable vertical or horizontal reactors for carrying out the process of the invention are equipped with stirrers for the aqueous medium to provide sufficient contact of gas phase monomers such as TFE for desirable reaction rates and uniform incorporation of comonomers if employed. The reactor preferably includes a cooling jacket surrounding the reactor so that the reaction temperature may be conveniently controlled by circulation of a controlled temperature heat exchange medium.

In a typical process employing the preferred polymerization agent comprising a branched fluoropolyether carboxylic acid or salt having a number average molecular weight of at least about 800 g/mol in combination with a branched fluoroether carboxylic acid or salt surfactant, the reactor is first charged with deionized and deaerated water of the polymerization medium and fluoropolyether acid or salt and fluorosurfactant are dispersed in the medium. The dispersing of the branched fluoropolyether carboxylic acid or salt having a number average molecular weight of at least about 800 g/mol in combination with a branched fluoroether carboxylic acid or salt surfactant is carried out as discussed above. For PTFE homopolymer and modified PTFE, paraffin wax as stabilizer is often added. A suitable procedure for PTFE homopolymer and modified PTFE includes first pressurizing the reactor with TFE. If used, the comonomer such as HFP or perfluoro (alkyl vinyl ether) is then added. A free-radical initiator solution such as ammonium persulfate solution is then added. For PTFE homopolymer and modified PTFE, a second initiator which is a source of succinic acid such as disuccinyl peroxide may be present in the initiator solution to reduce coagulum. Alternatively, a redox initiator system such as potassium permanganate/oxalic acid is used. The temperature is increased and, once polymerization begins, additional TFE is added to maintain the pressure. The beginning of polymerization is referred to as kick-off and is defined as the point at which gaseous monomer feed pressure is observed to drop substantially, for example, about 10 psi (about 70 kPa). Comonomer and/or chain transfer agent can also be added as the polymerization proceeds. For some polymerizations, additional monomers, initiator and or polymerization agent may be added during the polymerization.

Batch dispersion polymerizations can be described as proceeding in two phases. The initial period of the reaction can be said to be a nucleation phase during which a given number of particles are established. Subsequently, it can be said that a growth phase occurs in which the predominant action is polymerization of monomer on established particles with little or no formation of new particles. The transition from the nucleation to the growth phase of polymerization occurs smoothly, typically between about 4 and about 10 percent solids in for the polymerization of TFE.

After batch completion (typically several hours) when the desired amount of polymer or solids content has been achieved, the feeds are stopped, the reactor is vented and purged with nitrogen, and the raw dispersion in the vessel is transferred to a cooling vessel.

In a preferred process of the invention, polymerizing produces less than about 10 wt%, more preferably less than 3 wt%, even more preferably less than 1 wt%, most preferably less that about 0.5 wt% undispersed fluoropolymer (coagulum) based on the total weight of fluoropolymer produced.

For the production of PTFE fine powder, wet fluoropolymer, i.e., PTFE resin is isolated from the dispersion, usually by coagulation subsequent removal of the aqueous medium by filtration, and the PTFE is dried to produce fine powder. The drying step is described in more detail hereinafter.

The dispersion polymerization of melt-processible copolymers is similar except that comonomer in significant quantity is added to the batch initially and/or introduced during polymerization. Chain transfer agents are typically used in significant amounts to decrease molecular weight to increase melt flow rate. For melt-processible fluoropolymers used as molding resin, wet fluoropolymer resin is isolated from the dispersion, usually by coagulation subsequent removal of the aqueous medium by filtration. The fluoropolymer is dried as is described in more detail hereinafter then processed into a convenient form such as flake, chip or pellet for use in subsequent melt-processing operations.

The polymerization portion of the process of the invention may also be carried out as a continuous process in a pressurized reactor. A continuous process is especially useful for the manufacture of fluorocarbon elastomers.

Wet fluoropolymer resin isolated from the dispersion typically contains significant quantities of residual fluoroether carboxylic acids or salts. In accordance with the present invention, the wet fluoropolymer resin is heated both to remove water to produce a dry fluoropolymer resin and to decarboxylate residual fluoroether carboxylic acid or salt to produce a vapor of fluoroether byproduct. As is known in the art, any of a wide variety of commercial drying equipment can be used for drying the wet fluoropolymer resin such as tray driers, belt driers, etc. Preferably, a gas flow is provided during heating which provides an exhaust gas stream which carries water vapor and the vapor of fluoroether byproduct away from the fluoropolymer resin.

In accordance with preferred forms of the present invention, the resin is heated to a temperature of about 150°C to about 250°C, more preferably, about 160°C to about 200°C. As illustrated in the Decarboxylation Examples which follow, both branched and linear fluoroether carboxylic acids or salts decarboxylate upon heating but branched fluoroether carboxylic acids or salts which are employed in the process of the invention do so at a lower temperature as indicated by a lower Temperature of Maximum Decarboxylation. In addition, the Decarboxylation Half Life (t_{1/2}) at 200°C for branched fluoroether carboxylic acids or salts are significantly lower. Preferably, a branched fluoroether carboxylic acid or salt employed in accordance with the present invention has a Decarboxylation Half Life (t_{1/2}) measured as the ammonium salt at 200°C of less than about 30 minutes, more preferably, less than about 20 minutes, most preferably, less than about 10 minutes. In a preferred form of the invention, the fluoroether carboxylic acids or salts selected for polymerization and process conditions employed result in at least about 50% of the residual fluoroether carboxylic acid or salt in the wet fluoropolymer resin decarboxylating during heating. Preferably, at least about 65%, more preferably, 75%, and most preferably at least about 80% of the residual fluoroether carboxylic acid or salt in the wet fluoropolymer resin decarboxylates during heating.

Since the salt form of fluoroether carboxylic acid typically is more readily decarboxylated that the acid form, higher rates and higher fluoroether byproduct yields are promoted by isolating the wet fluoropolymer resin at high pH so that the residual fluoroether carboxylic acid is primarily in salt form. This can be accomplished by addition of ammonium carbonate to the dispersion prior to mechanical coagulation to isolate the wet fluoropolymer resin.

Decarboxylation results in the evolution of carbon dioxide and produces hydrofluoroether byproducts of the branched fluoroether carboxlic acids or salts. For example, when preferred fluoroether carboxylic acids or salts of the following Formula II above, the hydrofluoroether byproducts are the corresponding 2-hydrofluoroethers of the following structure shown in Formula IV:

CF₃-CF₂-CF₂-O-(-CFCF₃-CF₂-O-)ₙ-CFHCF₃ (IV)

Compounds of this structure are known and are useful industrially for various uses, e.g., as solvents. A compound of this structure when n=0 is available commercially from the DuPont Company as "E1 Stable Fluid" or "Freon® E-1 ". Compounds of this structure when n=1 and n=2 are available from DuPont as "Freon® E-2" and "Freon® E-3", respectively. In addition, the 2-hydrofluorethers of formula IV can be fluorinated to produce perfluoropolyethers which are useful for a variety of uses, e.g., as lubricants such as the oils and greases sold under the trademark Krytox® by the DuPont Company.

In preferred forms of the invention, it is desirable that the byproducts of the fluoroether carboxylic acids be in vapor form at the drier temperature employed. As has been discussed for the preferred polymerization agent in accordance with the invention comprising a mixture of branched fluoropolyether carboxylic acids or salts having a number average molecular weight of at least about 800 g/mol in combination with a branched fluoroether carboxylic acid or salt surfactant, it is preferred that fractions having values for n of 13 or greater in Formula IV for mixtures of preferred branched fluoropolyether carboxylic acids be limited, e.g., not more than 40% by weight of the mixture. This significantly decreases fluoroether carboxylic acid or salt or decarboxylation byproducts thereof in the dried fluoropolymer resin. In a preferred form of the invention, the dry fluoropolymer resin contains less than about 250 ppm residual fluoroether carboxylic acid or salt or decarboxylation byproducts thereof, more preferably, less than about 150 ppm, most preferably less than about 100 ppm. Preferred fluoropolymer resin typically contains about 25 ppm to about 100 ppm residual fluoroether carboxylic acid or salt or decarboxylation byproducts thereof.

In accordance with the invention, the vapor of fluoroether byproduct is captured. This preferably is accomplished by passing the exhaust gas stream through a bed of adsorbent particles. By the term "adsorbent particles" in connection with the present invention is meant particles that are capable of physically adsorbing the fluorinated surfactant by any mechanism of physical adsorption. In a preferred embodiment of the invention, the vapor of fluoroether byproduct present in the exhaust stream is captured in a bed of activated carbon. If desired, the temperature and relative humidity of the exhaust stream are adjusted before contacting the bed of adsorbent particles to improve performance and bed life. Preferably, for activated carbon, the temperature of the gas stream is adjusted to about 40°C to about 120°C. The relative humidity is preferably kept below 75%, more preferably below about 60%. Relative humidity below about 10% typically does not confer additional benefit. The temperature from the drier can be controlled with an appropriate heat exchanger. Relative humidity can be controlled by employing suitable air flow at the selected temperature.

In a preferred form of the invention, the fluoroether byproduct that is captured is recovered. When a bed of activated carbon is employed, recovery is advantageously accomplished by thermal desorbtion the fluoroether byproduct from the adsorbent particles. Steam stripping is a suitable method. Solvent extraction followed by solvent separation, e.g., distillation, can also be employed for recovery. As discussed previously, recovered fluoroether byproducts are useful as solvents and as intermediates in the manufacture of other products.

Alternatively, captured fluoroether byproducts can be disposed of in an environmentally sound manner. For example, when an activated carbon bed is employed, activated carbon saturated with fluoroether byproduct can be burned.

If desired, an aqueous scrubber can be employed in addition to the bed of absorbent particles to capture fluoroether carboxylic acid or salt which does not decarboxylate during drying. The aqueous medium in the scrubber can be water or water containing an appropriate base, e.g., 10% wt% NaOH. This scrubber can be employed in the exhaust gas stream either before or after the bed of absorbent particles. If the scrubber is after the bed of absorbent particles, the absorbent particles also absorb some of the fluoroether carboxylic acids or salts. If recovery is accomplished by thermal desorption, the fluoroether carboxylic acids or salts will usually be decarboxylated during such recovery and converted to fluoroether byproducts.

If the scrubber is employed before the bed of absorbent particles, it is believed that less fluoroether carboxylic acids or salts will be decarboxylated compared to providing the scrubber in the stream after the absorbent particle bed because the scrubber captures nearly all of the fluoroether carboxylic acids or salts which are present in the exhaust stream prior to reaching the activated carbon cartridge. The scrubber may also result in capture of fluoroether byproducts since vapor may be condensed when contacted with the aqueous scrubbing medium depending upon the molecular weight of the fluoroether byproduct and the temperature of the scrubbing medium. A decanter can be used if desired for separation of the fluoroether byproducts if present in sufficient quantity to form a separate phase from the aqueous scrubbing medium. When the scrubber is employed prior to the bed of absorbent particles, it may also be desirable to cool the exhaust stream to condense excess water which the exhaust stream picks up in the scrubber and which may interfere with the absorbent bed. Prior to entering the absorbent bed but after the cooler for humidity control, a heater may be employed to heat the exhaust stream to a more desirable temperature (and relative humidity) for effective absorption of the fluoroether byproducts on the absorbent bed.

### TEST METHODS

The melting point (Tm) and glass transition temperature (Tg) of copolymers is measured by Differential Scanning Calorimetry according to the procedure of ASTM D 4591. PTFE homopolymer melting point, the melting point the first time the polymer is heated, also referred to as the first heat, is determined by differential scanning calorimetry (DSC) by the method of ASTM D-4591-87. The melting temperature reported is the peak temperature of the endotherm on first melting.

Standard specific gravity (SSG) (PTFE) is measured by the method of ASTM D-4895.

Comonomer content (PPVE or HFP) is measured by FTIR according to the method disclosed in U.S. Patent No. 4,743,658, col. 5, lines 9-23.

Comonomer content (PDD) is measured by IR by comparing the absorbance ratio at 2404 cm⁻¹ to 1550 cm⁻¹ to a calibration curve

Melt flow rate (MFR) is measured according to ASTM D-1238 at the temperature which is standard for the specific copolymer.

Raw dispersion particle size (RDPS) is measured by photon correlation spectroscopy using a Microtrac®Nanotrac Particle Size Analyzer.

Temperature of Complete Weight Loss is measured by Thermal Gravimetric Analysis with Infrared analyzer (TGA/IR) using a TA Q500 TGA coupled with a Nicolet FT-IR instrument.

Temperature of Maximum Decarboxylation is measured by Thermal Gravimetric Analysis with Infrared analyzer (TGA/IR) using a TA Q500 TGA coupled with a Nicolet FT-IR instrument. The temperature at which CO₂ evolution is highest is the Temperature of Maximum Decarboxylation.

Decarboxylation Half Life (t_{1/2}) at 200°C is measured by headspace Gas Chromatography/Mass Selective Detector analysis (GC/MSD) by monitoring the formation of the corresponding hydrofluoroether while heating the ammonium salt of the fluoroether carboxylic acid at 200°C. For the analysis, a kinetic plot is constructed using known concentrations of the fluoroether ammonium salt in separate headspace vials heated in an oven at 200°C. At selected time intervals, the headspace of each vial is analyzed by GC/MSD with the amount of hydrofluoroether detected and estimated to construct the kinetic plot.

Number and Weight Average Molecular Weight and n in Formula (I) for Fluoropolyether Carboxylic Acid or Salt - The weight average molecular weight of the mixture of fluoropolyether acids or salts is determined by gas chromatography (GC) on an instrument equipped with either a flame ionization detector (FID) or a mass selective detector (MSD). Gas chromatography is suitably conducted on a chromatographic instrument such as an Agilent Model 6890. The fluoropolyether carboxylic acid or salt is first dissolved in a suitable solvent such as 2,3-dihydrodecafluoropentane (Vertrel® XF available from the DuPont Company) prior to GC injection. Typically, the fluoropolyether acid or salt with a concentration of less than 1 % in the solvent are injected to a GC injector port which has a typical injector temperature of ≥300°C. For the purpose of this test method, the high temperature in the injector port will thermally convert the injected fluoropolyether acid or salt to the corresponding hydrofluoropolyether (Formula VI). The retention time of the different oligomers can be obtained using reference standards of known Formula VI composition. The area of each oligomer (GC area %) is measured and used to calculate the weight average molecular weight. The number average molecular weight is calculated from weight average molecular weight using standard formulas and is also independently measured using 19F NMR spectroscopy. Molecular weights are reported in this application as the carboxylic acid and not the converted hydrofluoroether compound. Average n in Formula (I) is derived from the weight average molecular weight.

The amount of fluoropolyether carboxylic acid or salt having a molecular weight above or below a certain level, e.g., 2500 g/mol or greater (or n being 13 or greater in Formula (I) is determined from the same data used for weight average molecular weight. Since GC area % values are a good approximation of weight percent, GC area % values for the oligomers in the range of interest can be added together to determine the weight percent of oligomers in the range of interest.

Special selective ion monitoring (SIM) detection mode of the GC using a mass selector detector is utilized for the quantification for perfluoropolyether acid or salt of Formula (I) in which n=1 and calibration standards of known concentrations are prepared.

Residual Analysis - Using a 2,3-dihydrodecafluoropentane solvent (Vertrel® XF available from the DuPont Company), multiple solvent extractions of polymer samples are performed at elevated temperature and the resulting extracted solvent is analyzed for total fluoropolyether acids or salts and by-products thereof by GC with either FID or MSD as described above by comparison to calibration standards of hydrofluoropolyether (Formula VI) in the same solvent.

### EXAMPLES

### EXAMPLES - DECARBOXYLATION OF BRANCHED AND LINEAR FLUOROETHER CARBOXYLIC ACID SALTS

The Temperature of Complete Weight Loss, the Temperature of Maximum Decarboxylation, and Decarboxylation Half Life (t_{1/2}) at 200°C are measured for ammonium salts of selected branched and linear fluoroether carboxylic acids. The results are reported in Table 1 below.

The data in Table 1 shows that the Temperature of Maximum Decarboxylation and the Decarboxylation Half Life (t_{1/2}) at 200°C are significantly lower for branched fluoroether carboxylic acids than for linear fluoroether carboxylic acids. The data illustrates that, in a drier for wet fluoropolymer which operates at approximately 200°C, substantially more of the residual branched fluoroether carboxylic acid salt will be decarboxylated than linear fluoroether carboxylic acid salt at the same drier temperature.

**Table 1**

| **Compound** | **Linear/ Branched** | **Temp/C Complete Wt Loss*** | **Temp/C Max Decarboxylation**** | **t_{1/2 (min)} at 200°C in air***** |
|---|---|---|---|---|
| C₃F₇OCF(CF₃)CO₂NH₄ | Branched | 197 | 207 | ~5 |
| C₂F₅OCF(CF₃)CO₂NH₄ | Branched | 189 | 178 | ~10 |
| C₂F₅O(CF₂)₃CO₂NH₄ | Linear | 180 | 240 | ~90 |
| C₂F₅O(CF₂)₅CO₂NH₄ | Linear | 179 | 240 | |
| C₃F₇O(CF₂)₂CO₂NH₄ | Linear | 190 | 247 | -90 |
| C₃F₇O(CF₂)₃CO₂NH₄ | Linear | 180 | 240 | |
| C₃F₇O(CF₂)₅CO₂NH₄ | Linear | 181 | 240 | |
| * temp at which all samples were either evaporated or decomposed (decarboxylated). | | | | |
| ** temp at which maximum amount of CO₂ was observed during the TGA experiments. | | | | |
| *** half life of surfactants to undergo decarboxylation at 200C in air. | | | | |

### EXAMPLES - POLYMERIZATION AND ABATEMENT OF FLUOROETHER CARBOXYLIC ACID OR SALT

### Polymerization Agent Components

Branched fluoroether carboxylic acid is employed having the formula CF₃CF₂CF₂OCF(CF₃)COOH (referred to as dimer acid or DA) which is converted to the ammonium salt in the example which follows (referred to as dimer acid salt or DAS).

A mixture of fluoropolyether carboxylic acids is employed (referred to as PFPEA), having the structure of Formula (II) (Y⁺ is H). The acids are converted to ammonium salts in the examples which follow.

The molecular weight distribution of the mixture of fluoropolyether carboxylic acids used in the example is listed in Table 1A. Average values listed in the table are based on number average molecular weight with the exception of the column labeled weight average molecular weight.

**Table 1A - Molecular Weight Distribution of PFPEA Mixture**

| | **# Avg MW** | **Wt Avg MW** | **Avg n** | **ppm n=1** | **%n ≤ 4** | **% n ≥ 13** | **% n ≥ 16** | **% n=3 to n=13** |
|---|---|---|---|---|---|---|---|---|
| **PFPEA** | 1556 | 1669 | 7.4 | 10 | 5.069 | 3.726 | 1.257 | 97.484 |

The ammonium hydroxide used is a 30 wt% aqueous solution (wt% calculated as NH₄OH).

Polymerization agent mixtures 1 and 2 are prepared as follows resulting in water clear or nearly water clear mixtures. The amounts of PFPEA and DA indicated in Table 2A in Example 1 below are combined in a 100 mL glass jar and vigorously stirred for ~5 to 30 minutes. The indicated amount of 30% ammonium hydroxide solution (NH₄OH) was slowly added to the PFPEA/DA mixture while cooling in a water bath to produce a concentrate. The resulting concentrate was slowly poured into a 4L glass beaker containing the indicated amount of rapidly stirring deionized (DI) water to provide a water clear or nearly water clear mixture.

### Example 1

The process of the invention is illustrated in the polymerization of polytetrafluoroethylene (PTFE) homopolymer employing a mixture of branched fluoroethers, one component being a perfluoromonoether carboxylic acid is employed having the formula CF₃CF₂CF₂OCF(CF₃)COOH (DA) and a perfluoropolyether carboxylic acid mixture described above and identified in Table 2A below as PFPEA.

A cylindrical, horizontal, water-jacketed, paddle-stirred, stainless steel reactor having a length to diameter ratio of about 1.5 and a water capacity of 10 gallons (37.9 L) is charged with 40.6 pounds (18.4 kg) of demineralized water, 600 g of paraffin wax, 0.05 g of the ethoxylated alcohol Tomadol^{®} 23-1, 4.3 g of succinic acid, and 15 mL of a 0.02 m/v % aqueous oxalic acid solution. While agitating at 46 rpm, the contents of the reactor are heated to 65°C, and the reactor is evacuated and purged three times with tetrafluoroethylene (TFE). An aqueous mixture comprised of polymerization agent mixture 1 (see Table 1A) and deionized water (1552.2 g) is added. TFE is added until the pressure is 400 psig (2.9 MPa). Then, 240 mL of an aqueous initiator solution comprised of 0.036 g of KMnO₄ and 0.017 g of ammonium phosphate is added at the rate of 80 mL/min. When this addition is completed, 2375 mL of an aqueous mixture comprised of polymerization agent mixture 2 (see Table 2A) and deionized water (2312.7 g) is added at a rate of 64 mL/min and additional initiator solution is added at a rate of 5 mL/min. TFE is added at a rate sufficient to maintain 2.9 MPa. After 3 lbs (6.6 kg) of TFE has been added following initial pressurization, the temperature is raised to 72°C. After 14.6 lbs (32.1 kg) of TFE has been added following initial pressurization, initiator solution addition is stopped. After 17.6 lbs of TFE has been added following initial pressurization, the temperature is raised to 80°C. After a total of 24 lbs (52.8 kg) of TFE has been fed following initial pressurization, TFE addition is stopped and the reactor is vented. The contents of the reactor are discharged and the supernatant wax is removed. Solids content of the dispersion is 36.89 wt% and the raw dispersion particle size (RDPS) is 230.3 nm. The dispersion is diluted to 12% solids and coagulated in the presence of ammonium carbonate under vigorous agitation to produce wet PTFE resin.

For drying of the wet PTFE resin and abatement of the fluoroether carboxylic acids or salts, 300 g of the wet PTFE resin is transferred into a 500 ml glass vessel equipped with a heating jacket and ports for air inflow and exhaust outflow. Airflow (0.5 l/min) is begun and the vessel is heated to 150°C which is an adequate temperature to rapidly dry the wet PTFE and to decarboxylate the fluoroether carboxylic acids or salts. The exhaust from the vessel passes through a heat exchanger for cooling to approximately 60°C and 50% relative humidity and then enters a metal cartridge filled with activated carbon which is capable of absorbing the byproducts of decarboxylation the fluoroether carboxylic acids or salts, i.e., 2-hydrofluoroethers. The exhaust stream leaving the activated carbon cartridge is directed under the surface a 10% aqueous NaOH solution contained in a 250 ml bottle cooled with an ice water bath which serves as a scrubber for fluoroether carboxylic acids or salts which do not decarboxylate. Heating is continued for about 2.5 hours at which time the PTFE fine powder appears to be completely dry.

The activated carbon cartridge is removed from the system, one end is plugged and the other end connected to a condenser and collection vessel. The cartridge is heated ~150°C to drive off, i.e., thermally desorb, fluoroether byproducts captured by the cartridge and fluoroether byproducts are collected as a single phase oily liquid in the collection vessel. In addition, the sodium hydroxide scrubber solution is analyzed for fluoroether carboxylic acid or salt. Based on the weight of fluoroether byproducts recovered from the cartridge compared to the total fluoroether carboxylic acid or salt determined to be present in the scrubber solution, ~80% of the branched fluoroether carboxylic acids or salts are decarboxylated and captured in this process employed in this example.

Properties of the resulting PTFE fine powder are reported in Table 2B.

**Table 2A**

| Example | Polym. Agent Mixtures | Polym. Agent Mixture Type | PFPE Quantity (g) | DA Quantity (g) | NH₄OH Quantity (g) |
|---|---|---|---|---|---|
| Ex 1 | 1 | Precharge | 2.7 | 26.5 | 6.0 |
| | 2 | Pump | 6.4 | 64.0 | 10.5 |

**Table 2B**

| Example | Reaction Time (min) | Solids (wt %) | RDPS (nm) | Wet Coagulum (g) | SSG |
|---|---|---|---|---|---|
| Ex 1 | 113 | 35.99% | 237.5 | 470 | 2.176 |

### Example 2

The 300 g of wet PTFE resin is made and dried as in Example 1 except, during drying, the exhaust stream from the 0.5 liter glass vessel first enters the NaOH scrubbing solution. After leaving the scrubbing solution, the exhaust stream is passed though a condenser to remove excess water and then the stream is passed though a heat exchanger to increase its temperature to ~60°C (which results in a relative humidity of ~50%) prior to entering the activated carbon cartridge.

Based on the weight of fluoroether byproducts recovered from the cartridge compared to the total fluoroether carboxylic acid or salt determined to be present in the scrubber solution, ~76% of the branched fluoroether carboxylic acids or salts are decarboxylated and captured in this process employed in this example. It is believed that less fluoroether carboxylic acids or salts are decarboxylated in this example compared to Example 1 because the scrubber captures nearly all of the fluoroether carboxylic acids or salts which are present in the exhaust stream prior to reaching the activated carbon cartridge. In Example 1, the activated carbon cartridge also absorbs some of the fluoroether carboxylic acids or salts which are subsequently decarboxylated during thermal desorption to recover the fluoroether byproducts.

The polymer properties reported in Table 2B are not affected by the alternate abatement procedure employed in Example 2.

## Claims

1. A process for making fluoropolymer resin comprising:
polymerizing at least one fluorinated monomer in an aqueous medium containing initiator and polymerization agent comprising fluoroether carboxylic acid or salt thereof to form an aqueous dispersion of particles of fluoropolymer, said fluoroether carboxylic acid or salt thereof having the formula:
[R¹-O-L-COO⁻] Y⁺
wherein:
R¹ is a linear, branched or cyclic partially or fully fluorinated aliphatic group which may contain ether linkages;
L is a branched partially or fully fluorinated alkylene group which may contain ether linkages; and
Y⁺ is hydrogen, ammonium or alkali metal cation;
isolating wet fluoropolymer resin containing residual fluoroether carboxylic acid or salt from said aqueous medium;
heating said wet fluoropolymer resin to remove water to produce a dry fluoropolymer resin and to decarboxylate said residual fluoroether carboxylic acid or salt to produce a vapor of fluoroether byproduct; and
capturing said vapor of fluoroether byproduct.

2. The process of claim 1 wherein L is a branched fully fluorinated alkylene group which may contain ether linkages.

3. The process of claim 1 wherein L is -CF(CF₃)-.

4. The process of claim 1 wherein said fluoroether carboxylic acid or salt comprises a compound or a mixture of compounds with R¹ being CF₃-CF₂-CF₂-O-(-CFCF₃-CF₂-O-)ₙ and n is 0-35, L is -CF(CF₃)-.

5. The process of claim 1. wherein Y⁺ is hydrogen or ammonium.

6. The process of claim 5 wherein n is 0.

7. The process of claim 1 wherein at least 50% of said residual fluoroether carboxylic acid or salt decarboxylates during said heating to produce said vapor of fluoroether byproduct.

8. The process of claim 1 wherein said heating is performed at a temperature of 150°C to 250°C.

9. The process of claim 1 wherein said vapor of fluoroether byproduct is captured in a bed of adsorbent particles.

10. The process of claim 9 wherein said vapor of fluoroether byproduct is captured in a bed of activated carbon.

11. The process of claim 9 wherein said captured fluoroether byproduct is recovered.

12. The process of claim 11 wherein said recovery comprises thermally desorbing said fluoroether byproduct from said adsorbent particles.

13. The process of claim 1 wherein said fluoroether carboxylic acid or salt has a decarboxylation half-life in ammonium salt form of less than 30 minutes at 200°C.

14. The process of claim 1 further comprising providing a gas flow to said wet fluoropolymer resin during said heating to carry water vapor and said vapor of fluoroether byproduct away from said fluoropolymer resin.

## Patentansprüche

1. Verfahren zur Herstellung von Fluorpolymerharz, umfassend:
das Polymerisieren mindestens eines fluorierten Monomers in einem wässrigen Medium, das Initiator und Polymerisationsmittel enthält, umfassend Fluorethercarbonsäure oder Salz davon, um eine wässrige Dispersion von Fluorpolymerteilchen zu bilden, wobei die Fluorethercarbonsäure oder das Salz davon die Formel:
[R¹-O-L-COO⁻] Y⁺
aufweist, wobei
R¹ eine lineare, verzweigte oder cyclische, teilweise oder vollständig fluorierte aliphatische Gruppe ist, die Etherverknüpfungen enthalten kann;
L eine verzweigte, teilweise oder vollständig fluorierte Alkylengruppe ist, die Etherverknüpfungen enthalten kann; und
Y⁺ Wasserstoff, Ammonium oder ein Alkalimetallkation ist;
das Isolieren von nassem Fluorpolymerharz, das restliche Fluorethercarbonsäure oder Salz enthält, von dem wässrigen Medium;
das Erhitzen des nassen Fluorpolymerharzes, um Wasser zu entfernen, um ein trockenes Fluorpolymerharz herzustellen und die restliche Fluorethercarbonsäure oder das Salz zu decarboxylieren, um einen Dampf des Fluorether-Nebenprodukts herzustellen; und
das Auffangen des Dampfes von Fluorether-Nebenprodukt.

2. Verfahren nach Anspruch 1, wobei L eine verzweigte vollständig fluorierte Alkylengruppe ist, die Etherverknüpfungen enthalten kann.

3. Verfahren nach Anspruch 1, wobei L -CF(CF₃)- ist.

4. Verfahren nach Anspruch 1, wobei die Fluorethercarbonsäure oder das Salz eine Verbindung oder eine Mischung von Verbindungen umfasst, bei denen R¹ CF₃-CF₂-CF₂-O-(-CFCF₃-CF₂-O)ₙ ist und n 0 - 35 beträgt, L -CF(CF₃)- ist.

5. Verfahren nach Anspruch 1, wobei Y⁺ Wasserstoff oder Ammonium ist.

6. Verfahren nach Anspruch 5, wobei n 0 beträgt.

7. Verfahren nach Anspruch 1, wobei mindestens 50 % der restlichen Fluorethercarbonsäure oder des Salzes während des Erhitzens decarboxyliert werden, um Dampf von Fluorether-Nebenprodukt herzustellen.

8. Verfahren nach Anspruch 1, wobei das Erhitzen bei einer Temperatur von 150 °C bis 250 °C durchgeführt wird.

9. Verfahren nach Anspruch 1, wobei der Dampf von Fluorether-Nebenprodukt in einem Bett von Adsorptionsmittelteilchen aufgefangen wird.

10. Verfahren nach Anspruch 9, wobei der Dampf von Fluorether-Nebenprodukt in einem Bett von Aktivkohle aufgefangen wird.

11. Verfahren nach Anspruch 9, wobei das aufgefangene Fluorether-Nebenprodukt wiedergewonnen wird.

12. Verfahren nach Anspruch 11, wobei das Wiedergewinnen das thermische Desorbieren des Fluorether-Nebenprodukts aus den Adsorptionsmittelteilchen umfasst.

13. Verfahren nach Anspruch 1, wobei die Fluorethercarbonsäure oder das Salz eine Decarboxylierungs-Halbwertzeit in Ammoniumsalzform von weniger als 30 Minuten bei 200 °C aufweist.

14. Verfahren nach Anspruch 1, des Weiteren das Bereitstellen einer Gasströmung zu dem nassen Fluorpolymerharz während des Erhitzens umfassend, um Wasserdampf und den Dampf von Fluorether-Nebenprodukt von dem Fluorpolymerharz hinweg zu führen.

## Revendications

1. Procédé de fabrication d'une résine de polymère fluoré comprenant:
la polymérisation d'au moins un monomère fluoré dans un milieu aqueux contenant un initiateur et un agent de polymérisation comprenant de l'acide fluoroéther carboxylique ou sel de celui-ci pour former une dispersion aqueuse de particules de polymère fluoré, ledit acide fluoroéther carboxylique ou sel de celui-ci ayant la formule:
[R¹-O-L-COO⁻]Y⁺
dans laquelle:
R¹ est un groupe aliphatique linéaire, ramifié ou cyclique partiellement ou entièrement fluoré qui peut contenir des liaisons éther;
L est un groupe alcylène ramifié partiellement ou entièrement fluoré qui peut contenir des liaisons éther; et
Y⁺ est un atome d'hydrogène, un ammonium ou un cation de métal alcalin;
l'isolement de la résine de polymère fluoré humide contenant de l'acide fluoroéther carboxylique résiduel ou sel dudit milieu aqueux;
le chauffage de ladite résine de polymère fluoré humide pour retirer l'eau afin de produire une résine de polymère fluoré sèche et pour décarboxyler ledit acide fluoroéther carboxylique résiduel ou sel pour produire une vapeur de sous-produit éther fluoré; et
la capture de ladite vapeur du sous-produit d'éther fluoré.

2. Procédé selon la revendication 1, dans lequel L est un groupe alcylène ramifié entièrement fluoré qui peut contenir des liaisons éther.

3. Procédé selon la revendication 1, dans lequel L est -CF(CF₃)-.

4. Procédé selon la revendication 1, dans lequel ledit acide fluoroéther carboxylique ou sel comprend un composé ou un mélange de composés avec R¹ étant CF₃-CF₂-CF₂-O-(-CFCF₃-CF₂-O-)ₙ et n est 0-35, L est -CF(CF₃)-.

5. Procédé selon la revendication 1, dans lequel Y⁺ est un atome d'hydrogène ou un ammonium.

6. Procédé selon la revendication 5, dans lequel n a la valeur de 0.

7. Procédé selon la revendication 1, dans lequel au moins 50 % dudit acide fluoroéther carboxylique résiduel ou sel est décarboxylé durant ledit chauffage pour produire ladite vapeur de sous-produit éther fluoré.

8. Procédé selon la revendication 1, dans lequel ledit chauffage est effectué à une température de 150°C à 250°C.

9. Procédé selon la revendication 1, dans lequel ladite vapeur de sous-produit d'éther fluoré est capturée dans un lit de particules adsorbantes.

10. Procédé selon la revendication 9, dans lequel ladite vapeur de sous-produit d'éther fluoré est capturée dans un lit de charbon actif.

11. Procédé selon la revendication 9, dans lequel ledit sous-produit d'éther fluoré capturé est récupéré.

12. Procédé selon la revendication 11, dans lequel ladite récupération comprend la désorption thermique dudit sous-produit d'éther fluoré à partir desdites particules adsorbantes.

13. Procédé selon la revendication 1, dans lequel ledit acide fluoroéther carboxylique ou sel a une demi-vie de décarboxylation sous une forme de sel d'ammonium inférieure à 30 minutes à 200°C.

14. Procédé selon la revendication 1, comprenant en outre la fourniture d'un écoulement gazeux vers ladite résine de polymère fluoré humide durant ledit chauffage pour transporter la vapeur d'eau et ladite vapeur de sous-produit éther fluoré hors de ladite résine de polymère fluoré.
